# EUROPÄISCHE PATENTSCHRIFT

(11) **EP 1 926 728 B1**
(45) Veröffentlichungstag und Bekanntmachung des Hinweises auf die Patenterteilung: **05.04.2017**
(21) Anmeldenummer: 06777166.7
(22) Anmeldetag: 05.09.2006
(51) Int. Cl.: C07D 413/14, C07D 273/01, C07C 259/18, C07C 259/10, A01N 43/88

(54) **OXDIAZIN-SUBSTITUIERTE ARYLAMIDE**
OXADIAZINE-SUBSTITUTED ARYLAMIDES
ARYLAMIDES A SUBSTITUTION OXADIAZINE

(30) Priorität: 15.09.2005 DE 102005044108
(43) Veröffentlichungstag der Anmeldung: 04.06.2008
(73) Patentinhaber: Bayer Intellectual Property GmbH, 40789 Monheim am Rhein (DE)
(72) Erfinder: KRÜGER, Bernd-Wieland, 51467 Bergisch Gladbach (DE); HENSE, Achim, 51379 Leverkusen (DE); ALIG, Bernd, 53639 Königswinter (DE); FISCHER, Rüdiger, 50259 Pulheim (DE); FUNKE, Christian, 42799 Leichlingen (DE); GESING, Ernst, Rudolf, 40699 Erkrath (DE); MALSAM, Olga, 51503 Rösrath (DE); DREWES, Mark, Wilhelm, 40764 Langenfeld (DE); ARNOLD, Christian, 40764 Langenfeld (DE); LÜMMEN, Peter, 65510 Idstein (DE); SANWALD, Erich, 24159 Kiel (DE)
(74) Vertreter: BIP Patents
(86) Internationale Anmeldenummer: PCT/EP2006/008637
(87) Internationale Veröffentlichungsnummer: WO 2007/031213

(56) Entgegenhaltungen:
- WO-A-00/15622
- WO-A-01/49664
- WO-A-97/00866
- WO-A-02/094791
- WO-A-03/016304
- JP-A- 2005 132 727

## Beschreibung

Die vorliegende Erfindung betrifft neue Dioxazin- und Oxdiazin-substituierte Arylamide, mehrere Verfahren zu ihrer Herstellung und ihre Verwendung als Wirkstoffe, insbesondere ihre Verwendung als Schädlingsbekämpfungsmittel.

Es ist bereits bekannt, dass bestimmte substituierte 5,6-Dihydro-1,4,2-dioxazine (vgl. JP2005132727) insektizide und acarizide Eigenschaften besitzen.

Es ist ebenfalls bekannt, dass bestimmte Arylamide (WO 03/016304) und Anthranilamide (NL 9202078, WO 01/70671, WO 02/094791, JP 03212,834, WO 03/015519, WO 03/016284, WO 03/015518, WO 03/015519, WO 03/024222, WO 03/016282, WO 03/016283, WO 03/062226, WO 03/027099, WO 2004/027042, WO 2004/033468) insektizide Eigenschaften besitzen. Die Verbindungen der WO03/016304A beschreiben Arylamide, die in ortho Position zur Amidgruppe mit einem 5 oder 6-gliedrigen aromatischen oder nichtaromatischen Heterocyclus mit maximal 4 Stickstoffatomen und maximal 2 Sauerstoff- oder Schwefelatomen substituiert ist. Ein Tetrahyrodioxazin- oder oxdiazinring ist in diese Anmeldung nicht erwähnt.

Die Wirksamkeit dieser Stoffe ist gut, lässt aber in manchen Fällen zu wünschen übrig.

Es wurden nun neue Dioxazin- und Oxdiazin-substituierte Arylamide der Formel (I) 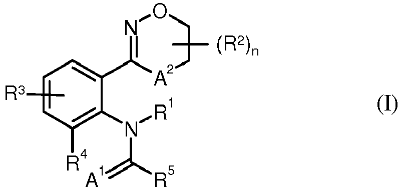 gefunden, in welcher
wobei
- A¹: für Sauerstoff steht,
- A2: für Amino steht,
- R¹: für Wasserstoff, Methyl, Cyclopropyl, Cyanomethyl, Methoxymethyl, Methylthiomethyl, Methylsulfinylmethyl oder Methylsulfonylmethyl steht,

- R²: unabhängig voneinander für gegebenenfalls einfach oder mehrfach, gleich oder verschieden substituiertes C₁-C₄-Alkyl, wobei die Substituenten unabhängig voneinander ausgewählt sein können aus Halogen, Cyano, Nitro, C₁-C₄-Alkoxy und C₁-C₄-Haloalkoxy, steht,
- n: für 0 steht,
- R³: für Wasserstoff, C₁-C₄-Alkyl, C₁-C₂-Haloalkyl, Halogen, Cyano oder C₁-C₂-Haloalkoxy steht,
- R⁴: für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsilyl steht,
- R⁵: für ein Pyrazol- oder Pyrrolring der Reihe R⁵-3 bis R⁵-8 steht, wobei jedes R⁵ mit R⁶ substituiert ist und gegebenenfalls mit R⁷ oder R⁸ oder sowohl R⁷ als auch R⁸ substituiert sein kann,

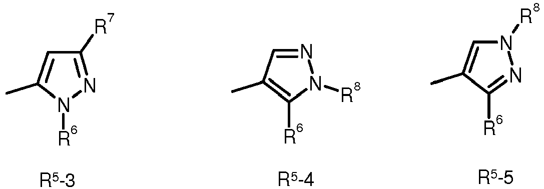

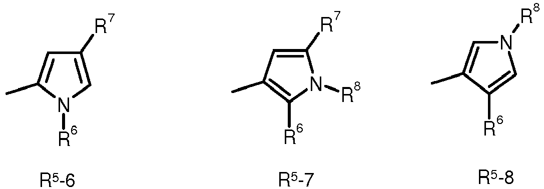

- R⁶: für 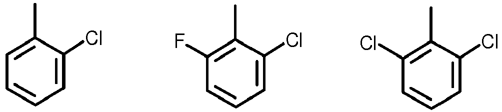 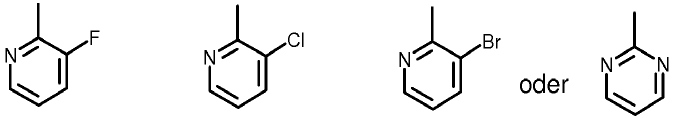 steht,
- R⁷: für Wasserstoff, Halogen oder C₁-C₄-Haloalkyl steht,
- R⁸: für Wasserstoff, C₁-C₄-Alkyl oder C₁-C₄-Haloalkyl steht,
sowie deren N-Oxide und Salze.

Schließlich wurde gefunden, dass die erfindungsgemäßen Verbindungen der Formel (I) sehr gute insektizide Eigenschaften besitzen und sich sowohl im Pflanzenschutz als auch im Materialschutz zur Bekämpfung unerwünschter Schädlinge, wie Insekten, verwenden lassen.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Anthranilamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R¹: steht ganz besonders bevorzugt für Wasserstoff.
- R²: steht ganz besonders bevorzugt für C₁-C₄-Alkyl.
- R³: steht ganz besonders bevorzugt für Wasserstoff, Methyl, Trifluormethyl, Cyano, Fluor, Chlor, Brom, Iod oder Trifluormethoxy.
- R³: steht insbesondere bevorzugt für Wasserstoff, Chlor , Brom oder Iod.
- R³: steht ausserdem insbesondere bevorzugt für Cyano.
- R⁴: steht ganz besonders bevorzugt für Methyl, Fluor, Chlor, Brom oder Iod.
- R⁴: steht insbesondere bevorzugt für Methyl oder Chlor.
- R⁵: steht ganz besonders bevorzugt für einen Pyrazolring R⁵-3.
- R⁶: steht ganz besonders bevorzugt für

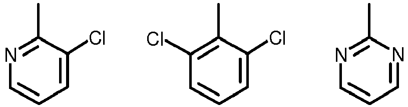

- R⁷: steht ganz besonders bevorzugt für Trifluormethyl, Chlor oder Brom
- R⁸: steht besonders bevorzugt, für Wasserstoff, C₁-C₄-Alkyl oder C₁-C₄-Haloalkyl.
- X¹: steht bevorzugt für N oder CCl.
- X²: steht besonders bevorzugt für N oder CCl

Hervorgehoben sind Verbindungen der Formel (1-1) 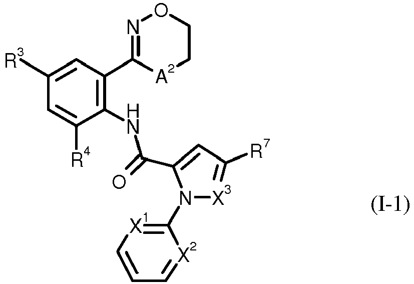 in welcher A², X¹, X², X³, R³, R⁴ und R⁷ die angegebenen allgemeinen, bevorzugten, besonders bevorzugten, ganz besonders bevorzugten bzw. insbesondere bevorzugten Bedeutungen haben.
- X³: steht für N oder CH.

Durch Halogen substituierte Reste, z.B. Haloalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Bevorzugt, besonders bevorzugt oder ganz besonders bevorzugt sind Verbindungen, welche jeweils die unter bevorzugt, besonders bevorzugt oder ganz besonders bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Weiterhin wurde gefunden, dass man Dioxazin- und Oxdiazin-substituierte Arylamide der Formel (I) nach einem der folgenden Verfahren erhält.

Dioxazin- und Oxdiazin-substituierte Arylamide der Formel (I-a) 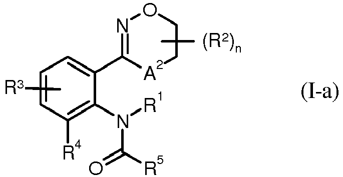 in welcher A², R¹, R², R³, R⁴ und R⁵ die oben angegebenen Bedeutungen haben, werden erhalten, indem man
(A) Dioxazin- und Oxdiazin-substituierte Aniline der Formel (II) 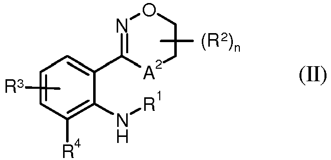 in welcher A², R¹, R², R³ und R⁴ die oben angegebenen Bedeutungen haben,
   mit Carbonsäurechloriden der Formel (III) 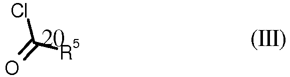 in welcher R⁵ die oben angegebenen Bedeutungen hat, in Gegenwart eines Säurebindemittels umsetzt.

Dioxazin- und Oxdiazin-substituierte Arylthioamide der Formel (I-b) 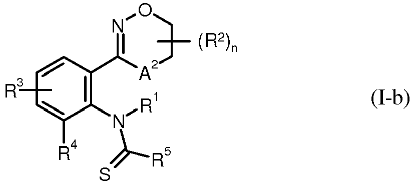 in welcher A², R¹, R², R³, R⁴ und R⁵ die oben angegebenen Bedeutungen haben, werden erhalten, indem man
(B) Dioxazin- und Oxdiazin-substituierte Arylamide der Formel (I-a) 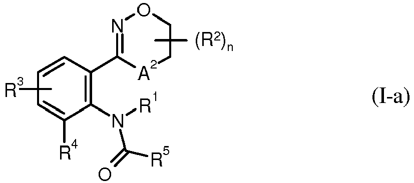 in welcher A², R¹, R², R³, R⁴ und R⁵ die oben angegebenen Bedeutungen haben,
   mit einem Schwefelungsreagenz umsetzt.

### Erläuterung der Verfahren und Zwischenprodukte

### Verfahren (A)

Verwendet man beispielsweise 2,4-Dichlor-6-(5,6-dihydro-[1,4,2]dioxazin-3-yl)-phenylamin und 3-Trifluormethyl-1-(3-chlorpyridin-2-yl)-1H-pyrazol-5-carbonylchlorid als Ausgangsstoffe, so kann der Verlauf des erfindungsgemäßen Verfahrens (A) durch das folgende Formelschema veranschaulicht werden.

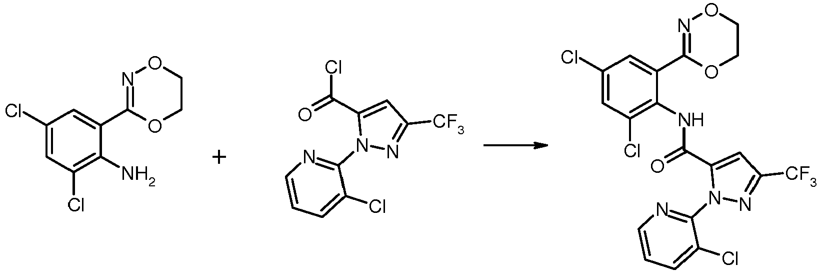

Die bei der Durchführung des erfindungsgemäßen Verfahrens (A) als Ausgangsstoffe benötigten Dioxazin- und Oxdiazin-substituierte Aniline sind durch die Formel (II) allgemein definiert. In dieser Formel (II) stehen A², R¹, R², R³ und R⁴ bevorzugt, besonders bevorzugt, ganz besonders bevorzugt bzw. insbesondere bevorzugt für diejenigen Bedeutungen, die bereits im Zusammenhang mit der Beschreibung der erfindungsgemäßen Stoffe der Formel (I) als bevorzugt, besonders bevorzugt usw. für diese Reste genannt wurden.

Das erfindungsgemäße Verfahren (A) wird in Gegenwart eines Säurebindemittels durchgeführt. Hierzu eignen sich alle für solche Kupplungsreaktionen üblichen anorganischen oder organischen Basen. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, Diisopropylethylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU). Ebenso lassen sich gegebenenfalls polymer-gestützte Säurebindemittel, wie z.B. polymer-gebundenes Diisopropylamin und polymer-gebundenes Dimethylaminopyridin einsetzen.

Das erfindungsgemäße Verfahren (A) kann gegebenenfalls in Gegenwart eines für solche Reaktionen üblichen inerten organischen Verdünnungsmittel durchgeführt werden. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser. Besonders bevorzugt verwendbar sind Toluol, Tetrahydrofuran und N,N-Dimethylformamid.

Die Reaktionstemperaturen können bei der Durchführung des erfindungsgemäßen Verfahrens (A) in einem größeren Bereich variiert werden. Im Allgemeinen arbeitet man bei Temperaturen von 0°C bis 150°C, vorzugsweise bei Temperaturen von 20°C bis 100°C.

Dioxazin- und Oxdiazin-substituierte Aniline der Formel (II) sind neu. Für R¹ ≠ H lassen sie sich beispielsweise herstellen, indem man
(C) Dioxazin-substituierte Aniline der Formel (II-a) oder Oxdiazin-substituierte Aniline der Formel (II-b), 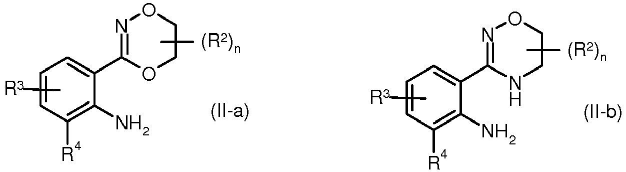 in welcher R², R³ und R⁴ die oben angegebenen Bedeutungen haben, mit einem Alkylierungsmittel (z. B. R¹-Halogenid) in Gegenwart einer Base (z. B. Kaliumcarbonat) in Gegenwart eines Verdünnungsmittels (z. B. Tetrahydrofuran oder N,N-Dimethylformid) oder zuerst in einer Kondensationsreaktion (z. B. mit einem R¹-Aldehyd) in Gegenwart eines Verdünnungsmittels (z. B. Toluol) und anschließend mit einem Reduktionsmittel (z. B. Natriumcyanoborhydrid) in Gegenwart eines Verdünnungsmittels (z. B. Methanol) umsetzt.

Für Verbindungen der Formel (II) mit R¹ = H ist Verfahren (C) unnötig. Hier werden die Verbindungen der Formel (II-a) oder (II-b) direkt als Edukt im Verfahren (A) eingesetzt.

Dioxazin-substituierte Aniline der Formel (II-a) lassen sich beispielsweise herstellen, indem man
(D) 2-Amino-*N*-(2-hydroxyethoxy)-benzamide der Formel (IV) 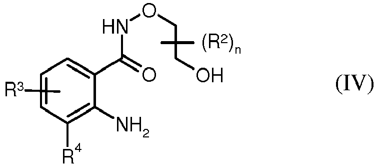 in welcher R², R³ und R⁴ die oben angegebenen Bedeutungen haben, mit einem Aktivierungsmittel (z.B. Thionylchlorid) sowie gegebenenfalls in Gegenwart eines Verdünnungsmittels umsetzt.

Die Reaktionstemperaturen können bei der Durchführung des erfindungsgemäßen Verfahrens (D) in einem größeren Bereich variiert werden. Im Allgemeinen arbeitet man bei Temperaturen von 0°C bis 150°C, vorzugsweise bei Temperaturen von 60°C bis 80°C.

2-Amino-*N*-(2-hydroxyethoxy)-benzamide der Formel (IV) lassen sich beispielsweise herstellen, indem man
(E) 2-Aminobenzoesäureester der Formel (V) 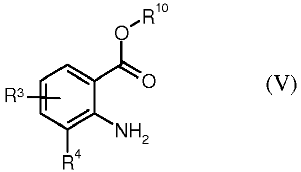 in welcher R³ und R⁴ die oben angegebenen Bedeutungen haben und R¹⁰ für C₁-C₄-Alkyl steht, mit 2-Aminooxyethanol-Derivaten der Formel (VI) 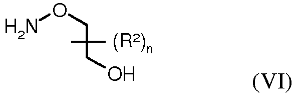 in welcher (R²)ₙ die oben angegebenen Bedeutungen haben in Gegenwart einer Base (z.B. Natriummethanolat oder Natriumethanolat) sowie gegebenenfalls in Gegenwart eines Verdünnungsmittels (z.B. Methanol oder Ethanol) umsetzt.

2-Aminobenzoesäureester der Formel (V) sind bekannt oder lassen sich nach bekannten Verfahren erhalten (vgl. z.B. E. A. Meyer, M. Furler, F. Diederich, R. Brenk, G. Klebe, Helv. Chim. Acta 2004, 87, 1333-1356).

2-Aminooxyethanol-Derivate der Formel (VI) sind bekannt oder lassen sich nach bekannten Verfahren erhalten (vgl. z.B. US 3184500, DE 2651083, DE 2820013).

Carbonsäurechloride der Formel (III) sind bekannt (vgl. z.B. WO 03/016284, WO 03/016304).

Oxdiazin-substituierte Aniline der allgemeinen Formel (II-b) lassen sich beispielsweise herstellen, indem man
(F) Oxdiazin-substituierte 2-Nitrophenyl-Derivate der Formel (VII) 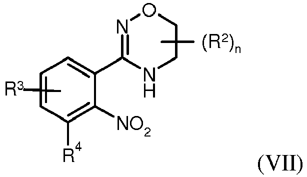 in welcher R², R³ und R⁴ die oben angegebenen Bedeutungen haben, mit einem Reduktionsmittel (z.B. Palladium auf Aktivkohle in Gegenwart von Wasserstoff) in Gegenwart eines Verdünnungsmittels (z. B. Ethanol) umsetzt.

Oxdiazin-substituierte Aniline der Formel (II-b), in welcher R³ für 4-Chlor, 4-Brom oder 4-Iod steht und R² und R4 die oben angegebenen Bedeutungen haben, lassen sich vorteilhaft herstellen, indem man
(G) Oxdiazin-substituierte Aniline der allgemeinen Formel (II-b') 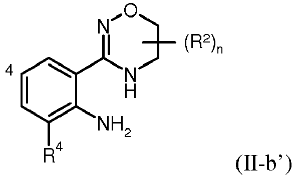 in welcher R², R⁴ und n die oben angegebenen Bedeutungen haben, mit einem Halogenierungsmittel (z.B. N-Chlorsuccinimid, N-Bromsuccinimid oder N-Iodsuccinimid) in Gegenwart eines Verdünnungsmittels (z. B. N,N-Dimethylformamid) umsetzt.

Die Reaktionstemperaturen können bei der Durchführung des erfindungsgemäßen Verfahrens (G) in einem größeren Bereich variiert werden. Im Allgemeinen arbeitet man bei Temperaturen von 0°C bis 150°C, vorzugsweise bei Temperaturen von 80°C bis 120°C.

Oxdiazin-substituierte 2-Nitrophenyl-Derivate der Formel (VII) lassen sich beispielsweise herstellen, indem man
(H) *N*-Alkoxy-2-nitrobenzamidin-Derivate der Formel (VIII) 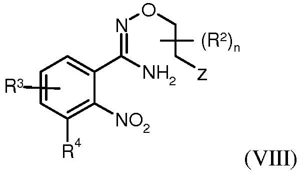 in welcher R², R³ und R⁴ die oben angegebenen Bedeutungen haben und Z für Chlor, Brom, Iod, Methylsulfonyl oder Tolylsulfonyl steht, mit einer Base (z. B. Natriumhydrid) in Gegenwart eines Lösungsmittels (z.B. Tetrahydrofuran, Dimethylacetamid oder N-Methylpyrrolidinon) umsetzt.

Die Reaktionstemperaturen können bei der Durchführung des erfindungsgemäßen Verfahrens (H) in einem größeren Bereich variiert werden. Im Allgemeinen arbeitet man bei Temperaturen von 0°C bis 150°C, vorzugsweise bei Temperaturen von 60°C bis 100°C.

*N*-Alkoxy-2-nitrobenzamidin-Derivate der Formel (VIII) lassen sich beispielsweise herstellen, indem man
(I) *N*-(2-Hydroxyethoxy)-2-nitro-benzamidin-Derivate der Formel (IX) 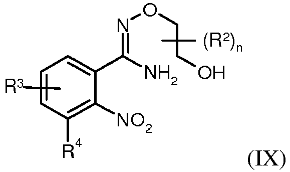 in welcher R², R³ und R⁴ die oben angegebenen Bedeutungen haben, mit einem Sulfonylchlorid (z.B. Methylsulfonsäurechloid oder Toluolsulfonsäurechlorid) oder einem Halogenierungsmittel (z.B. Thionylchlorid) gegebenenfalls unter Anwesenheit eines Lösungsmittels (z.B. Dichlormethan) und gegebenenfalls unter Anwesenheit einer Base (z.B. Triethylamin) umsetzt.

Die Reaktionstemperaturen können bei der Durchführung des erfindungsgemäßen Verfahrens (I) in einem größeren Bereich variiert werden. Im Allgemeinen arbeitet man bei Temperaturen von 0°C bis 150°C, vorzugsweise bei Temperaturen von 0°C bis 60°C.

*N*-(2-Hydroxyethoxy)-2-nitro-benzamidin-Derivate der Formel (IX) lassen sich beispielsweise herstellen, indem man
(J) 2-Nitrobenziminoester der Formel (X) 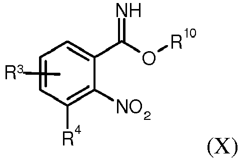 in welcher R³ und R⁴ die oben angegebenen Bedeutungen haben und R¹⁰ für C₁-C₄-Alkyl steht, mit 2-Aminooxyethanol-Derivaten der Formel (VI) 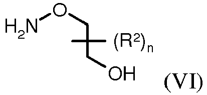 in welcher R² und n die oben angegebenen Bedeutungen haben in Gegenwart eines anorganischen Salzes (z.B. Ammoiumchlorid) sowie in Gegenwart eines Verdünnungsmittels (z.B. Methanol oder Ethanol) umsetzt.

Die Reaktionstemperaturen können bei der Durchführung des erfindungsgemäßen Verfahrens (J) in einem größeren Bereich variiert werden. Im Allgemeinen arbeitet man bei Temperaturen von 0°C bis 150°C, vorzugsweise bei Temperaturen von 20°C bis 60°C.

2-Nitrobenziminoester der Formel (X) sind bekannt oder lassen sich nach bekannten Verfahren erhalten (vgl. z.B. H. Okada, T. Koyanagi, N. Yamada, Chem. Pharm. Bull. 1994, 42, 57-61; EP 335408).

### Verfahren (B)

Verwendet man beispielsweise als 5-Brom-2-(3-chlorpyridin-2-yl)-2H-pyrazol-3-carbonsäure [2,4-dichlor-6-(5,6-dihydro-[1,4,2]dioxazin-3-yl)phenyl]amid als Ausgangsstoff und Lawsson's Reagenz als Schwefelungsmittel, so kann der Verlauf der erfindungsgemäßen Verfahrens (B) durch das folgende Formelschema veranschaulicht werden.

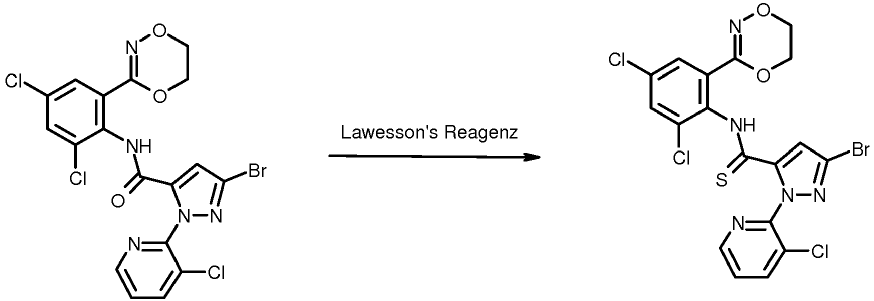

Die bei der Durchführung der erfindungsgemäßen Verfahrens (B) als Ausgangsstoffe benötigten Anthranilamide sind durch die Formeln (I-a) allgemein definiert. In der Formel (I-a) stehen A², R¹, R², R³, R⁴ und R⁵ bevorzugt, besonders bevorzugt, ganz besonders bevorzugt bzw. insbesondere bevorzugt für diejenigen Bedeutungen, die bereits im Zusammenhang mit der Beschreibung der erfindungsgemäßen Stoffe der Formel (I) als bevorzugt, besonders bevorzugt usw. für diese Reste genannt wurden.

Die Anthranilamide der Formeln (I-a) sind eine Untergruppe der Anthranilamide der Formel (I) und können nach den Verfahren (A) erhalten werden.

Als Schwefelungsreagenz können alle für solche Reaktionen üblichen Reagenzien verwendet werden. Vorzugsweise verwendbar sind Phosphorpentasulfid und Lawesson's Reagenz.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semi-penetrans, Xiphinema spp.

Die erfindungsgemäßen Verbindungen der Formel (I) zeichnen sich insbesondere durch starke Wirkung gegen Blattläuse (z.B. *Myzus persicae*), Schmetterlingsraupen (z.B. *Plutella xylostella, Spodoptera frugiperda, Spodoptera exigua, Heliothis armigera*) und Käferlarven (z.B. *Phaedon cochleariae*) aus.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch einoder mehrschichtiges Umhüllen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:

### Fungizide:

Inhibitoren der Nucleinsäure Synthese
   Benalaxyl, Benalaxyl-M, Bupirimat, Chiralaxyl, Clozylacon, Dimethirimol, Ethirimol, Furalaxyl, Hymexazol, Mefenoxam, Metalaxyl, Metalaxyl-M, Ofurace, Oxadixyl, Oxolinsäure
Inhibitoren der Mitose und Zellteilung
   Benomyl, Carbendazim, Diethofencarb, Ethaboxam, Fuberidazole, Pencycuron, Thiabendazol, Thiophanat-methyl, Zoxamid
Inhibitoren der Atmungskette Komplex I
   Diflumetorim
Inhibitoren der Atmungskette Komplex II
   Boscalid, Carboxin, Fenfuram, Flutolanil, Furametpyr, Furmecyclox, Mepronil, Oxycarboxin, Penthiopyrad, Thifluzamid
Inhibitoren der Atmungskette Komplex III
   Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
Entkoppler
   Dinocap, Fluazinam
Inhibitoren der ATP Produktion
   Fentinacetat, Fentinchlorid, Fentinhydroxid, Silthiofam
Inhibitoren der Aminosäure- und Proteinbiosynthese
   Andoprim, Blasticidin-S, Cyprodinil, Kasugamycin, Kasugamycinhydrochlorid Hydrat, Mepanipyrim, Pyrimethanil
Inhibitoren der Signal-Transduktion
   Fenpiclonil, Fludioxonil, Quinoxyfen
Inhibitoren der Fett- und Membran Synthese
   Chlozolinat, Iprodion, Procymidon, Vinclozolin
   Ampropylfos, Kalium-Ampropylfos, Edifenphos, Etridiazol, Iprobenfos (IBP), Isoprothiolan, Pyrazophos
   Tolclofos-methyl, Biphenyl
   lodocarb, Propamocarb, Propamocarb hydrochlorid, Propamocarb-Fosetylat
Inhibitoren der Ergosterol Biosynthese
   Fenhexamid,
   Azaconazol, Bitertanol, Bromuconazol, Cyproconazol, Diclobutrazol, Difenoconazol, Diniconazol, Diniconazol-M, Epoxiconazol, Etaconazol, Fenarimol, Fenbuconazol, Fluquinconazol, Flurprimidol, Flusilazol, Flutriafol, Furconazol, Furconazol-cis, Hexaconazol, Imazalil, Imazalilsulfat Imibenconazol, Ipconazol, Metconazol, Myclobutanil, Nuarimol, Oxpoconazol, Paclobutrazol, Penconazol, Pefurazoat Prochloraz, Propiconazol, Prothioconazol, Pyrifenox, Simeconazol, Tebuconazol, Tetraconazol, Triadimefon, Triadimenol, Triflumizol Triforin, Triticonazol, Uniconazol, Voriconazol, Viniconazol,
   Aldimorph, Dodemorph, Dodemorphacetat, Fenpropidin, Fenpropimorph, Spiroxamin, Tridemorph,
   Naftifin, Pyributicarb, Terbinafin
Inhibitoren der Zellwand Synthese
   Benthiavalicarb, Bialaphos, Dimethomorph, Flumorph, Iprovalicarb, Mandipropamid, Polyoxins, Polyoxorim, Validamycin A
Inhibitoren der Melanin Biosynthese
   Capropamid, Diclocymet, Fenoxanil, Phtalid, Pyroquilon, Tricyclazol
Resistenzinduktion
   Acibenzolar-S-methyl, Probenazol, Tiadinil
Multisite
   Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
Weitere Fungizide
   Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ferimzon, Flumetover, Flusulfamid, Fluopicolid, Fluoroimid, Fosetyl-Aluminium, Fosetyl-Caclcium, Fosetyl-Natrium, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorphenol und Salze, 2-Phenylphenol und Salze, Piperalin, Propanosin-Natrium, Proquinazid, Pyribencarb, Pyrrolnitrin, Quintozen, Tecloftalam, Tecnazen, Triazoxid, Trichlamid, Valiphenal , Zarilamid,
   2-(2-{[6-(3-Chlor-2-methylphenoxy)-5-fluorpyrimidin-4-yl]oxy}phenyl)-2-(methoxyimino)-N-methylacetamid,
   2-[[[[1-[3(1 Fluor-2-phenylethyl)oxy] phenyl] ethyliden]amino]oxy]methyl]-alpha-(methoxyimino)-N-methyl-alphaE-benzacetamid,
   cis-1-(4-Chlorphenyl)-2-(1H-1,2,4-triazol-1-yl)-cycloheptanol,
   1-[(4-Methoxyphenoxy)methyl]-2,2-dimethylpropyl-1H-imidazol-1-carbonsäure,
   2,3,5,6-Tetrachlor-4-(methylsulfonyl)-pyridin,
   2-Butoxy-6-iod-3-propyl-benzopyranon-4-on,
   2-Chlor-N-(2,3-dihydro-1,1,3-trimethyl-1H-inden-4-yl)-3-pyridincarboxamid,
   3,4,5-Trichlor-2,6-pyridindicarbonitril,
   3,4-Dichlor-N-(2-cyanophenyl)isothiazol-5-carboxamid (Isotianil)
   3-[5-(4-Chlorphenyl)-2,3-dimethylisoxazolidin-3-yl]pyridin,
   5-Chlor-6-(2,4,6-trifluorophenyl)-N-[(1R)-1,2,2-trimethylpropyl][1,2,4]triazolo[1,5-a]pyrimidin-7-amin,
   5-Chlor-7-(4-methylpiperidin-1-yl)-6-(2,4,6-trifluorophenyl)[1,2,4]triazolo[1,5-a]pyrimidin,
   5-Chlor-N-[(1R)-1,2-dimethylpropyl]-6-(2,4,6-trifluorophenyl) [1,2,4]triazolo[1,5-a]pyrimidin-7-amin,
   Methyl 2-[[[cyclopropyl[(4-methoxyphenyl) imino]methyl]thio]methyl]-.alpha.-(methoxymethylen)- benzacetat,
   Methyl 1-(2,3-dihydro-2,2-dimethyl-1H-inden-1-yl)-1H-imidazole-5-carboxylat,
   N-(3',4'-dichlor-5-fluorbiphenyl-2-yl)-3-(difluormethyl)-1-methyl-1 H-pyrazol-4-carboxamid,
   N-(3-Ethyl-3,5,5-trimethyl-cyclohexyl)-3-formylamino-2-hydroxy-benzamid,
   N-(4-Chlor-2-nitrophenyl)-N-ethyl-4-methyl-benzenesulfonamid,
   N-(4-chlorbenzyl)-3-[3-methoxy-4-(prop-2-yn-1-yloxy)phenyl]propanamid,
   N-[(4-chlorphenyl)(cyano)methyl]-3-[3-methoxy-4-(prop-2-yn-1-yloxy)phenyl]propanamid,
   N-(5-Brom-3-chlorpyridin-2-yl)methyl-2,4-dichlornicotinamid,
   N-[1-(5-Brom-3-chloropyridin-2-yl)ethyl]-2,4-dichloronicotinamid,
   (2S)-N-[2-[4-[[3-(4-chlorophenyl)-2-propinyl]oxy]-3-methoxyphenyl]ethyl]- 3-methyl-2-[(methylsulfonyl)amino]-butanamid,
   N-{(Z)-[(cyclopropylmethoxy) imino][6-(difluormethoxy)-2,3-difluorphenyl]methyl}-2-benzacetamid,
   N-{2-[1,1'-bi(cyclopropyl)-2-yl]phenyl}-3-(difluormethyl)-1-methyl-1H-pyrazol-4-carboxamid,
   N-{2-[3-Chlor-5-(trifluormethyl)pyridin-2-yl]ethyl}-2-(trifluoromethyl)benzamid,
   N-ethyl-N-methyl-N'-{2-methyl-5-(trifluormethyl)-4-[3-(trimethylsilyl)propoxy]phenyl}imidoformamid,
   O-[1-[(4-Methoxyphenoxy)methyl]-2,2-dimethylpropyl]-1H-imidazol- 1- carbothioic acid,
   2-Amino-4-methyl-N-phenyl-5-thiazolcarboxamid,
   2,4-Dihydro-5-methoxy-2-methyl-4-[[[[1-[3-(trifluoromethyl)-phenyl]-ethyliden]-amino]-oxy]-methyl]-phenyl]-3H-1,2,4-triazol-3-on (CAS Nr. 185336-79-2),
   N-(6-Methoxy-3-pyridinyl)-cyclopropan carboxamid,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren
   Carbamate,
      zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
   Organophosphate,
      zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, lodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/ -ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
Natrium-Kanal-Modulatoren / Spannungsabhängige Natrium-Kanal-Blocker
   Pyrethroide,
      zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1 R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1 R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1 R- isomer), Tralomethrin, Transfluthrin, ZXI 8901, Pyrethrins (pyrethrum)
   DDT
   Oxadiazine,
      zum Beispiel Indoxacarb
   Semicarbazon,
      zum Beispiel Metaflumizon (BAS3201)
Acetylcholin-Rezeptor-Agonisten/-Antagonisten
   Chloronicotinyle,
      zum Beispiel Acetamiprid, Clothianidin, Dinotefuran, Imidacloprid, Nitenpyram, Nithiazine, Thiacloprid, Thiamethoxam
   Nicotine, Bensultap, Cartap
Acetylcholin-Rezeptor-Modulatoren
   Spinosyne,
      zum Beispiel Spinosad
GABA-gesteuerte Chlorid-Kanal-Antagonisten
   Organochlorine,
      zum Beispiel Camphechlor, Chlordane, Endosulfan, Gamma-HCH, HCH, Heptachlor, Lindane, Methoxychlor
   Fiprole,
      zum Beispiel Acetoprole, Ethiprole, Fipronil, Pyrafluprole, Pyriprole, Vaniliprole
Chlorid-Kanal-Aktivatoren
   Mectine,
      zum Beispiel Abamectin, Emamectin, Emamectin-benzoate, Ivermectin, Lepimectin, Milbemycin
Juvenilhormon-Mimetika,
   zum Beispiel Diofenolan, Epofenonane, Fenoxycarb, Hydroprene, Kinoprene, Methoprene, Pyriproxifen, Triprene
Ecdysonagonisten/disruptoren
   Diacylhydrazine,
      zum Beispiel Chromafenozide, Halofenozide, Methoxyfenozide, Tebufenozide
Inhibitoren der Chitinbiosynthese
   Benzoylharnstoffe,
      zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
   Buprofezin
   Cyromazine
Inhibitoren der oxidativen Phosphorylierung, ATP-Disruptoren
   Diafenthiuron
   Organozinnverbindungen,
      zum Beispiel Azocyclotin, Cyhexatin, Fenbutatin-oxide
Entkoppler der oxidativen Phoshorylierung durch Unterbrechung des H-Protongradienten
   Pyrrole,
      zum Beispiel Chlorfenapyr
   Dinitrophenole,
      zum Beispiel Binapacyrl, Dinobuton, Dinocap, DNOC, Meptyldinocap
Seite-I-Elektronentransportinhibitoren
   METI's,
      zum Beispiel Fenazaquin, Fenpyroximate, Pyrimidifen, Pyridaben, Tebufenpyrad, Tolfenpyrad
   Hydramethylnon
   Dicofol
Seite-II-Elektronentransportinhibitoren
   Rotenone
Seite-III-Elektronentransportinhibitoren
   Acequinocyl, Fluacrypyrim
Mikrobielle Disruptoren der Insektendarmmembran
   Bacillus thuringiensis-Stämme
Inhibitoren der Fettsynthese
   Tetronsäuren,
      zum Beispiel Spirodiclofen, Spiromesifen,
   Tetramsäuren,
      zum Beispiel Spirotetramat, cis-3-(2,5-dimethylphenyl)-4-hydroxy-8-methoxy-1-azaspiro[4.5]dec-3-en-2-on
   Carboxamide,
      zum Beispiel Flonicamid
   Oktopaminerge Agonisten,
      zum Beispiel Amitraz
Inhibitoren der Magnesium-stimulierten ATPase,
   Propargite
   Nereistoxin-Analoge,
      zum Beispiel Thiocyclam hydrogen oxalate, Thiosultap-sodium
Agonisten des Ryanodin-Rezeptors,
   Benzoesäuredicarboxamide,
      zum Beispiel Flubendiamid
   Anthranilamide,
      zum Beispiel Rynaxypyr (3-bromo-N-{4-chloro-2-methyl-6-[(methylamino)carbonyl]phenyl}-1-(3-chloropyridin-2-yl)-1H-pyrazole-5-carboxamide)
Biologika, Hormone oder Pheromone
   Azadirachtin, Bacillus spec., Beauveria spec., Codlemone, Metarrhizium spec., Paecilomyces spec., Thuringiensin, Verticillium spec.
Wirkstoffe mit unbekannten oder nicht spezifischen Wirkmechanismen
   Begasungsmittel,
      zum Beispiel Aluminium phosphide, Methyl bromide, Sulfuryl fluoride
   Fraßhemmer,
      zum Beispiel Cryolite, Flonicamid, Pymetrozine
   Milbenwachstumsinhibitoren,
      zum Beispiel Clofentezine, Etoxazole, Hexythiazox Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene, Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die folgenden Herstellungs und Verwendungsbeispiele illustrieren die Erfindung, ohne sie zu beschränken.

### Herstellungsbeispiele

### Beispiel 1

### 1-(6-Chlor-2-pyridyl)-N-(2,4-dichlor-6-(3-(5,6-dihydro-1,4,2-dioxazinyl)-phenyl-3-trifluormethyl-1H-pyrazol-5-carboxamid (I-1-1):

Man legt 0,266g (0,857 mmol) 6-Chlor-2-(3-trifluormethyl-5-chlorcarbonyl-pyrazolyl)-pyridin in 15 ml Toluol unter Argon vor. Es werden 0,192g 3-(3,5-Dichlor-2-amino-phenyl)-5,6-dihydro-1,4,2-dioxin, 0,047g (1,6-Diazabicyclo[5.4.0]undec-7-en(1,5-5)) und 0,185g (2,337 mmol) Pyridin zugegeben. Nach 3 h Rühren unter Rückfluß kühlt man ab, gießt das Reaktionsgemisch in 200 ml Wasser und extrahiert dreimal mit je 100 ml Essigsäureethylester. Es wird über Natriumsulfat getrocknet, am Rotationsverdampfer zur Trockene eingeengt.

Der Rückstand wird über Chromatographie an Kieselgel (Cyclohexan/Essig-säureethylester = 3 : 1) gereinigt.

Ausbeute 0,26g (60% d. Th.)

Analog zu dem oben aufgeführten Beispielen (1-1-1) sowie der allgemeinen Beschreibung werden folgende Verbindungen der Formel (1-1) erhalten.

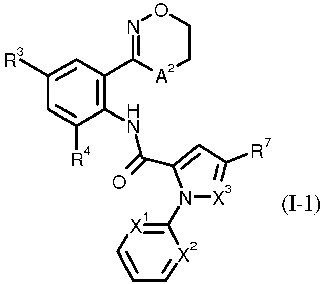

**Tabelle 1**

| **Bsp.-Nr.** | **A²** | **R³** | **R⁴** | **R⁷** | **X¹** | **X²** | **X³** | **logP** |
|---|---|---|---|---|---|---|---|---|
| I-1-2 | O | Cl | Cl | Cl | N | CCl | N | 3.07 |
| I-1-3 | O | H | CH₃ | CF₃ | N | CCl | N | 3.00 |
| I-1-4 | O | H | CH₃ | Br | N | CCl | N | 2.59 |
| I-1-5 | O | Cl | CH₃ | Br | N | CCl | N | 3.13 |
| I-1-6 | O | Cl | CH₃ | CF₃ | N | CCl | N | 3.50 |
| I-1-7 | O | H | CH₃ | Cl | N | CCl | N | 2.54 |
| I-1-8 | O | Cl | Cl | Br | N | CCl | N | 3.09 |
| I-1-9 | O | Cl | CH₃ | Cl | N | CCl | N | 3.08 |
| I-1-10 | O | Br | CH₃ | Cl | N | CCl | N | 3.18 |
| I-1-11 | O | Br | CH₃ | CF₃ | N | CCl | N | 3.58 |
| I-1-12 | O | Br | CH₃ | Br | N | CCl | N | 3.24 |
| I-1-13 | O | Cl | Cl | Br | CCl | CCl | N | 3.98 |
| I-1-14 | O | Cl | CH₃ | CF₃ | N | N | N | 2.79 |
| I-1-15 | O | Cl | Cl | CF₃ | N | N | N | 2.80 |
| I-1-16 | NH | H | CH₃ | CF₃ | N | CCl | N | 1.98 |
| I-1-17 | NH | H | CH₃ | Cl | N | CCl | N | 1.65 |
| I-1-18 | NH | Cl | CH₃ | CF₃ | N | CCl | N | 2.70 |
| I-1-19 | NH | Cl | CH₃ | Br | N | CCl | N | 2.25 |
| I-1-20 | NH | Cl | CH₃ | Cl | N | CCl | N | 2.25 |
| I-1-21 | O | Cl | Cl | Br | N | CCl | CH | 3.48 |
| I-1-22 | NH | I | CH₃ | Br | N | CCl | CH | 2.39 |
| I-1-23 | NH | I | CH₃ | Br | N | CCl | N | 2.21 |
| I-1-24 | NH | I | CH₃ | CF₃ | N | CCl | N | 2.66 |
| I-1-25 | O | Cl | CH₃ | Br | N | CCl | CH | 3.50 |
| I-1-26 | NH | I | CH₃ | Cl | N | CCl | N | 2.44 |
| I-1-27 | NH | H | Cl | Br | N | CCl | N | 1.89 |
| I-1-28 | NH | H | CH₃ | Br | N | CCl | CH | 1.83 |
| I-1-29 | NH | Br | CH₃ | CF₃ | N | CCl | N | 2.80 |
| I-1-30 | NH | Br | CH₃ | Cl | N | CCl | N | 2.35 |
| I-1-31 | NH | Br | CH₃ | Br | N | CCl | N | 2.37 |
| I-1-32 | NH | CN | CH₃ | Br | N | CCl | CH | 1.90 |

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung mit der LC-MS erfolgt im sauren Bereich bei pH 2,7 mit 0,1 % wässriger Ameisensäure und Acetonitril (enthält 0,1% Ameisensäure) als Eluenten; linearer Gradient von 10% Acetonitril bis 95% Acetonitril

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-max-Werte wurden an Hand der UV-Spektren von 200 nm bis 400 nm in den Maxima der chromatographischen Signale ermittelt.

### Herstellung von Ausgangsstoffen der Formel (II-a)

### Beispiel 2

### 3-(3,5-Dichlor-2-aminophenyl)-5,6-dihydro-1,4,2-dioxazin:

Man legt bei 20 °C 30 ml Thionylchlorid vor und gibt unter Rühren in 3 Portionen 3g 3,5-Dichlor-2-amino-N-(2-hydroxyethoxy)-benzamid zu. Es wird 12 h bei 70 ° C nachgerührt. Danach kühlt man ab und gießt das Reaktionsgemisch vorsichtig auf Eiswasser. Man extrahiert zweimal mit je 150 ml Dichlormethan, trocknet über Natriumsulfat und engt am Rotationsverdampfer zur Trockene ein.

Reinigung über Kieselgelchromatographie Cyclohexan/Essigsäureethylester = 10 : 1.

Ausbeute: 2g (62% d. Th.)

### Herstellung von Ausgangsstoffen der Formel (IV)

### Beispiel 3

### 3,5-Dichlor-2-amino-N-(2-hydroxyethoxy)benzamid:

Man legt 9g (40,9 mmol) 3,5-Dichlor-2-aminobenzoesäure-methylester und 6,3g (81,8 mmol)2-(Aminooxy)-ethanol in 90 ml Methanol vor und tropft bei 20 °C 22,1 g (122,7 mmol) Natriummethylat ,als 30%ige Lösung in Methanol, zu. Es wird über Nacht bei 50 °C nachgerührt. Man kühlt ab, gießt auf 400 ml Wasser und stellt mit 1 N Salzsäure auf pH 3. Es wird dreimal mit je 150 ml Essigsäureethylester extrahiert, über Natriumsulfat getrocknet und am Rotationsverdampfrer eingeengt.

Reinigung über Kieselgelchromatographie Cyclohexan/Essigsäureethylester = 3 : 1, dann Cyclohexan/Essigsäureethylester = 1 : 1.

Ausbeute: 4 g (30% d. Th.)

### Herstellung von Ausgangsstoffen der Formel (IIb)

### Beispiel 4

### 2-(5,6-Dihydro-4H-[1,2,4]oxadiazin-3-yl)-6-methyl-phenylaniin:

3 g (13,6 mmol) 3-(3-Methyl-2-nitrophenyl)-5,6-dihydro-4*H*-[1,2,4]oxadiazin werden in 40 ml Ethanol gelöst und mit 0,3 g Pd/C (5%ig) versetzt. Anschließend wird 72 h bei 21°C unter 3 bar Wasserstoff gerührt. Die Reaktionsmischung wird dann filtriert und Ethanol im Vakuum abdestilliert.

Ausbeute: 2,5 g

### Herstellung von Ausgangsstoffen der Formel (VII)

### Beispiel 5

### 3-(3-Methyl-2-nitrophenyl)-5,6-dihydro-4H-[1,2,4]oxadiazin:

1,4 g (5,4 mmol) 3-Methyl-*N*-(2-chlorethoxy)-2-nitrobenzamidin werden in 45 ml 1-Methyl-2-pyrrolidon vorgelegt und langsam mit 0,18 g (6 mmol) Natriumhydrid (80 %ig) versetzt. Es wird 12 h bei 100° C gerührt, abgekühlt, auf Wasser gegossen und mit Essigsäureethylester extrahiert. Nach trocknen über Natriumsulfat wird das Lösungsmittels im Vakuum abdestilliert.

Ausbeute: 0,75 g

### Herstellung von Ausgangsstoffen der Formel (VIII)

### Beispiel 6

### 3-Methyl-N-(2-chlorethoxy)-2-nitrobenzamidin:

4,75 (19,9 mmol) 3-Methyl-N-(2-hydroxyethoxy)-2-nitrobenzamidin werden 3 h bei 60°C in 45 ml (617 mmol) Thionylchlorid gerührt. Anschließend wird abgekühlt und vorsichtig auf Wasser gegossen. Man extrahiert mit Methylenchlorid, trocknet über Natriumsulfat und destilliert im Vakuum ab. Der Rückstand wird über Kieselgel chromatographiert (Cyclohexan:Essigsäureethylester = 4:1).

Ausbeute: 4,1 g

### Herstellung von Ausgangsstoffen der Formel (IX)

### Beispiel 7

### 3-Methyl-N-(2-hydroxyethoxy)-2-nitrobenzamidin:

3,4 g (17,5 mmol) 3-Methyl-2-Nitrobenziminomethylester und 2,7 g (35 mmol) 2-Amino-oxyethanol werden in 40 ml Ethanol vorgelegt. Man fügt ca. 150 mg Ammoniumchlorid hinzu und rührt weitere 12 h bei 40°C. Anschließend wird auf 300 ml Wasser gegossen und drei mal mit je 100 ml Essigsäureethylester extrahiert, über Natriumsulfat getrocknet und anschließend das organische Lösungsmittel im Vakuum abdestilliert.

Ausbeute: 4,3 g (99 % d. Theorie)

### Anwendungsbeispiele

### Beispiel Nr. 1

### Myzus persicae - Test

| | |
|---|---|
| Lösungsmittel: | 1 % N-methylpyrolidon (NMP) |
| | 1 % Diacetonalkohol |
| | |
| Farbstoff: Brillantsulfoflavin zum Anfärben des Wassers | |

Zur Herstellung einer zweckmäßigen Wirkstoffzubereitung vermischt man den Wirkstoff mit den angegebenen Mengen Lösungsmittel und verdünnt das Konzentrat mit angefärbtem Wasser auf die gewünschte Konzentration.

Den *Myzus persicae* wird eine Wirkstoffzubereitung der gewünschten Konzentration zur Aufnahme zur Verfügung gestellt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: I-1-7, I-1-8, I-1-9

**Tabelle 2**

| **Myzus-Test** | | |
|---|---|---|
| Wirkstoff | Wirkstoffkonzentration in ppm | Abtötungsgrad nach 6-7^{d} in % |
| I-1-7 | 30 | 100 |
| I-1-8 | 30 | 100 |
| I-1-9 | 30 | 100 |

### Beispiel Nr. 2

### Aedes Aegypti - Test

**Farbstoff: Brillantsulfoflavin zum Anfärben des Wassers**

| | |
|---|---|
| Lösungsmittel: | 1 % N-methylpyrolidon (NMP) |
| | 1 % Diacetonalkohol |

Zur Herstellung einer zweckmäßigen Wirkstoffzubereitung vermischt man den Wirkstoff mit den angegebenen Mengen Lösungsmittel und verdünnt das Konzentrat mit angefärbtem Wasser auf die gewünschte Konzentration.

Die Larven (*Aedes aegypti*) werden mit einer Wirkstoffzubereitung der gewünschten Konzentration behandelt.

Nach der gewünschten Zeit wird die Wirkung in % bestimmt. Dabei bedeutet 100 %, dass alle Larven abgetötet wurden; 0 % bedeutet, dass keine Larven abgetötet wurden.

Bei diesem Test zeigt z. B. die folgende Verbindung der Herstellungsbeispiele gute Wirksamkeit: 1-1-8

**Tabelle 3**

| **Aedes Aegypti - Test** | | |
|---|---|---|
| Wirkstoff | Wirkstoffkonzentration in ppm | Abtötungsgrad nach 2-4^{d} in % |
| I-1-8 | 30 | 100 |

### Beispiel Nr. 3

### Diabrotica undecimpunctata (DIABUN)

**Farbstoff: Brillantsulfoflavin zum Anfärben des Wassers**

| | |
|---|---|
| Lösungsmittel: | 1 % N-methylpyrolidon (NMP) |
| | 1 % Diacetonalkohol |

Zur Herstellung einer zweckmäßigen Wirkstoffzubereitung vermischt man den Wirkstoff mit den angegebenen Mengen Lösungsmittel und verdünnt das Konzentrat mit angefärbtem Wasser auf die gewünschte Konzentration.

Die Eier (*Diabrotica undecimpunctata*) werden mit einer Wirkstoffzubereitung der gewünschten Konzentration behandelt.

Nach der gewünschten Zeit wird die Wirkung in % bestimmt. Dabei bedeutet 100 %, dass alle Tiere abgetötet wurden; 0 % bedeutet, dass keine Tiere abgetötet wurden.

Bei diesem Test zeigen z. B. die folgenden Verbindungen der Herstellungsbeispiele gute Wirksamkeit: I-1-5, I-1-8, I-1-9

**Tabelle 4**

| **Diabrotica undecimpunctata - Test** | | |
|---|---|---|
| Wirkstoff | Wirkstoffkonzentration in ppm | Abtötungsgrad nach 2-5^{d} in % |
| I-1-5 | 300 | 100 |
| I-1-8 | 300 | 100 |
| I-1-9 | 300 | 100 |

### Beispiel Nr. 4

### Heliothis virescens

**Farbstoff: Brillantsulfoflavin zum Anfärben des Wassers**

| | |
|---|---|
| Lösungsmittel: | 1 % N-methylpyrolidon (NMP) |
| | 1 % Diacetonalkohol |

Zur Herstellung einer zweckmäßigen Wirkstoffzubereitung vermischt man den Wirkstoff mit den angegebenen Mengen Lösungsmittel und verdünnt das Konzentrat mit angefärbtem Wasser auf die gewünschte Konzentration.

Die Eier (*Heliothis virescens*) Eier werden mit einer Wirkstoffzubereitung der gewünschten Konzentration behandelt.

Nach der gewünschten Zeit wird die Wirkung in % bestimmt. Dabei bedeutet 100 %, dass alle Tiere abgetötet wurden; 0 % bedeutet, dass keine Tiere abgetötet wurden.

Bei diesem Test zeigen z. B. die folgenden Verbindungen der Herstellungsbeispiele gute Wirksamkeit: I-1-7, I-1-8, I-1-9

**Tabelle 5**

| **Heliothis virescens -Test** | | |
|---|---|---|
| Wirkstoff | Wirkstoffkonzentration in ppm | Abtötungsgrad nach 6-7^{d} in % |
| I-1-7 | 300 | 100 |
| I-1-8 | 100 | 100 |
| I-1-9 | 300 | 100 |

### Beispiel Nr. 5

### Phaedon -Test

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae*) besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit: I-1-1, I-1-2, I-1-5, I-1-6, I-1-8, I-1-9, I-1-10, I-1-11, I-1-12, I-1-13, I-1-16, I-1-17, I-1-18,1-1-19,1-1-20,1-1-22,1-1-23,1-1-24,1-1-25,1-1-26,1-1-29,1-1-30,1-1-32,1-1-21,1-1-28

**Tabelle 6**

| **Phaedon -Test** | | |
|---|---|---|
| Wirkstoff | Wirkstoffkonzentration in g/ha | Abtötungsgrad nach 7^{d} in % |
| I-1-1 | 500 | 100 |
| I-1-2 | 100 | 100 |
| I-1-5 | 100 | 83 |
| I-1-6 | 100 | 100 |
| I-1-8 | 100 | 100 |
| I-1-9 | 100 | 100 |
| I-1-10 | 100 | 100 |
| I-1-11 | 100 | 100 |
| I-1-12 | 100 | 100 |
| I-1-13 | 100 | 100 |
| I-1-16 | 100 | 100 |
| I-1-18 | 100 | 100 |
| I-1-19 | 100 | 100 |
| I-1-20 | 100 | 100 |
| I-1-22 | 100 | 100 |
| I-1-23 | 100 | 100 |
| I-1-24 | 100 | 100 |
| I-1-25 | 100 | 83 |
| I-1-26 | 100 | 100 |
| I-1-29 | 100 | 100 |
| I-1-30 | 100 | 100 |
| I-1-32 | 100 | 100 |
| I-1-28 | 100 | 100 |
| I-1-21 | 100 | 100 |

### Beispiel Nr. 6

### Myzus-Test (MYZUPE Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: I-1-2, I-1-16, I-1-17, I-1-18, I-1-19, I-1-20, I-1-22, I-1-23, I-1-24, I-1-26, I-1-29, I-1-30

**Tabelle 7**

| **Myzus-Test (Spritzbehandlung)** | | |
|---|---|---|
| Wirkstoff | Wirkstoffkonzentration in g/ha | Abtötungsgrad nach 5^{d} in % |
| I-1-2 | 100 | 80 |
| I-1-16 | 100 | 100 |
| I-1-17 | 100 | 100 |
| I-1-18 | 100 | 100 |
| I-1-19 | 100 | 90 |
| I-1-20 | 100 | 100 |
| I-1-22 | 100 | 100 |
| I-1-23 | 100 | 90 |
| I-1-24 | 100 | 90 |
| I-1-26 | 100 | 100 |
| I-1-29 | 100 | 100 |
| I-1-30 | 100 | 100 |

### Beispiel Nr. 7

### Spodoptera frugiperda-Test ( SPODFR Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele gute Wirksamkeit: I-1-1, I-1-2, I-1-3, I-1-4, I-1-5, I-1-6, I-1-7, I-1-8, I-1-9, I-1-10, I-1-11, I-1-12, I-1-16, I-1-17, I-1-18, I-1-19, I-1-20, I-1-22, I-1-23, I-1-24, I-1-25, I-1-26, I-1-29, I-1-30, I-1-32, I-1-21, I-1-28

**Tabelle 8**

| **Spodoptera -Test (Spritzbehandlung)** | | |
|---|---|---|
| Wirkstoff | Wirkstoffkonzentration in g/ha | Abtötungsgrad nach 7^{d} in % |
| I-1-1 | 500 | 100 |
| I-1-2 | 100 | 100 |
| I-1-3 | 100 | 100 |
| I-1-4 | 100 | 100 |
| I-1-5 | 100 | 100 |
| I-1-6 | 100 | 100 |
| I-1-7 | 100 | 100 |
| I-1-8 | 100 | 100 |
| I-1-9 | 100 | 100 |
| I-1-10 | 100 | 100 |
| I-1-11 | 100 | 100 |
| I-1-12 | 100 | 100 |
| I-1-16 | 100 | 100 |
| I-1-17 | 100 | 100 |
| I-1-18 | 100 | 100 |
| I-1-19 | 100 | 100 |
| I-1-20 | 100 | 100 |
| I-1-22 | 100 | 100 |
| I-1-23 | 100 | 100 |
| I-1-24 | 100 | 100 |
| I-1-25 | 100 | 83 |
| I-1-26 | 100 | 100 |
| I-1-29 | 100 | 100 |
| I-1-30 | 100 | 100 |
| I-1-32 | 100 | 100 |
| I-1-28 | 100 | 100 |
| I-1-21 | 100 | 100 |

### Beispiel Nr. 8

### Spodoptera frugiperda -Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Maispflanzen (*Zea mays*) werden mit einer Wirkstoffzubereitung der gewünschten Konzentration angegossen und mit *Spodoptera frugiperda* Larven infiziert.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele gute Wirksamkeit: I-1-1, I-1-3, I-1-6, I-1-8, I-1-9, I-1-10, I-1-11

**Tabelle 9**

| **Spodoptera -Test (Gießbehandlung)** | | |
|---|---|---|
| Wirkstoff | Wirkstoffkonzentration in ppm | Abtötungsgrad nach 14^{d} in % |
| I-1-1 | 20 | 95 |
| I-1-3 | 20 | 95 |
| I-1-6 | 20 | 98 |
| I-1-8 | 20 | 95 |
| I-1-9 | 20 | 95 |
| I-1-10 | 20 | 90 |
| I-1-11 | 20 | 98 |
| I-1-17 | 20 | 98 |
| I-1-18 | 20 | 95 |
| I-1-19 | 20 | 95 |
| I-1-20 | 20 | 95 |

### Beispiel Nr. 9

### Spodoptera exigua-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Tauchen in die Wirkstoffzubereitung der gewünschten Konzentration behandelt und mit Raupen des Heerwurms (*Spodoptera exigua*) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt z. B. die folgende Verbindung der Herstellungsbeispiele gute Wirksamkeit: I-1-l1 I-1-6, I-1-10, I-1-11, I-1-16, I-1-17, I-1-18, I-1-19, I-1-20

**Tabelle 10**

| **Spodoptera-exigua-Test** | | |
|---|---|---|
| Wirkstoff | Wirkstoffkonzentration in ppm | Abtötungsgrad nach 7^{d} in % |
| I-1-1 | 20 | 100 |
| I-1-6 | 100 | 100 |
| I-1-10 | 20 | 100 |
| I-1-11 | 20 | 100 |
| I-1-16 | 20 | 100 |
| I-1-17 | 4 | 100 |
| I-1-18 | 100 | 100 |
| I-1-19 | 4 | 80 |
| I-1-20 | 4 | 100 |

### Beispiel Nr. 10

### Heliothis armigera - Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Sojabohnenblätter (*Glycine max*.) werden durch Tauchen in die Wirkstoffzubereitung der gewünschten Konzentration behandelt und mit Raupen des Baumwollkapselwurms (*Heliothis armigera*) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit: I-1-1, I-1-8, I-1-9, I-1-10, I-1-11, I-1-16, I-1-17, I-1-18, I-1-19, I-1-20

**Tabelle 11**

| **Heliothis armigera -Test** | | |
|---|---|---|
| Wirkstoff | Wirkstoffkonzentration in ppm | Abtötungsgrad nach 7^{d} in % |
| I-1-1 | 20 | 100 |
| I-1-8 | 20 | 100 |
| I-1-9 | 20 | 80 |
| I-1-10 | 20 | 100 |
| I-1-11 | 20 | 100 |
| I-1-16 | 20 | 100 |
| I-1-17 | 4 | 100 |
| I-1-18 | 20 | 100 |
| I-1-19 | 4 | 100 |
| I-1-20 | 4 | 100 |

### Beispiel Nr. 11

### Spodoptera exigua-Test; resistenter Stamm

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Tauchen in die Wirkstoffzubereitung der gewünschten Konzentration behandelt und mit Raupen des Heerwurms (*Spodoptera exigua*, resistenter Stamm) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit: I-1-1, I-1-6, I-1-10, I-1-11, I-1-17, I-1-18, I-1-19

**Tabelle 12**

| **Spodoptera exigua-Test; resistenter Stamm** | | |
|---|---|---|
| Wirkstoff | Wirkstoffkonzentration in ppm | Abtötungsgrad nach 7^{d} in % |
| I-1-1 | 100 | 100 |
| I-1-6 | 20 | 100 |
| I-1-10 | 20 | 100 |
| I-1-11 | 20 | 80 |
| I-1-17 | 4 | 100 |
| I-1-18 | 100 | 100 |
| I-1-19 | 20 | 100 |

### Beispiel Nr. 12

### Plutella-Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Tauchen in die Wirkstoffzubereitung der gewünschten Konzentration behandelt und mit Raupen der Kohlschabe (*Plutella xylostella*) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt z. B. die folgende Verbindung der Herstellungsbeispiele gute Wirksamkeit: I-1-1, I-1-6, I-1-11, I-1-16, I-1-17, I-1-18, I-1-19, I-1-20

**Tabelle 13**

| **Plutella-Test** | | |
|---|---|---|
| Wirkstoff | Wirkstoffkonzentration in ppm | Abtötungsgrad nach 7^{d} in % |
| I-1-1 | 100 | 100 |
| I-1-6 | 20 | 100 |
| I-1-11 | 20 | 80 |
| I-1-16 | 100 | 100 |
| I-1-17 | 4 | 100 |
| I-1-18 | 20 | 100 |
| I-1-19 | 0,8 | 100 |
| I-1-20 | 4 | 100 |

### Beispiel Nr. 13

### Myzus persicae -Test; systemischeBehandlung

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Wasser gemischt. Die angegebene Konzentration bezieht sich auf die Wirkstoffmenge pro Volumeneinheit Wasser (mg/l = ppm). Man füllt das behandelte Wasser in Gefäße mit einer Erbsenpflanze (*Pisum sativum*), anschließend wird mit der Grünen Pfirsichblattlaus (*Myzus persicae*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindung der Herstellungsbeispiele gute Wirksamkeit: 1-1-16,1-1-18,1-1-19

**Tabelle 14**

| **Myzus persicae -Test; systemische Behandlung** | | |
|---|---|---|
| Wirkstoff | Wirkstoffkonzentration in ppm | Abtötungsgrad nach 6^{d} in % |
| I-1-16 | 20 | 100 |
| I-1-18 | 20 | 95 |
| I-1-19 | 20 | 90 |

### Beispiel Nr. 14

### Myzus-Test; oral;

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit allen Stadien der Grünen Pfirsichblattlaus (*Myzus persicae*) besetzt, durch saugen an der Wirkstoffzubereitung der gewünschten Konzentration wird behandelt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: I-1-1, I-1-16, I-1-22, I-1-23, I-1-24

**Tabelle 15**

| **Myzus persicae -Test; oral** | | |
|---|---|---|
| Wirkstoff | Wirkstoffkonzentration in ppm | Abtötungsgrad nach 5^{d} in % |
| I-1-16 | 100 | 100 |
| I-1-22 | 100 | 100 |
| I-1-23 | 100 | 100 |
| I-1-24 | 100 | 100 |

### Beispiel Nr. 15

### Aphis gossypii -Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter (*Gossypium hirsutum*), die stark von der Baumwollblattlaus (*Aphis gossypii*) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindungen der Herstellungsbeispiele gute Wirksamkeit: I-1-19, I-1-20

**Tabelle 16**

| **Aphis gossypii-Test** | | |
|---|---|---|
| Wirkstoff | Wirkstoffkonzentration in ppm | Abtötungsgrad nach 6^{d} in % |
| I-1-19 | 100 | 80 |
| I-1-20 | 100 | 80 |

## Patentansprüche

1. Verbindungen der Formel (I) 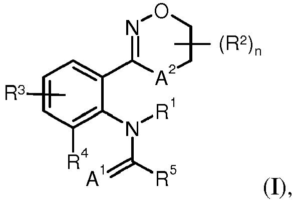 wobei
A¹ für Sauerstoff steht,
A2 für Amino steht,
R¹ für Wasserstoff, Methyl, Cyclopropyl, Cyanomethyl, Methoxymethyl, Methylthiomethyl, Methylsulfinylmethyl oder Methylsulfonylmethyl steht,
R² unabhängig voneinander für gegebenenfalls einfach oder mehrfach, gleich oder verschieden substituiertes C₁-C₄-Alkyl, wobei die Substituenten unabhängig voneinander ausgewählt sein können aus Halogen, Cyano, Nitro, C₁-C₄-Alkoxy und C₁-C₄-Haloalkoxy, steht,
n für 0 steht,
R³ für Wasserstoff, C₁-C₄-Alkyl, C₁-C₂-Haloalkyl, Halogen, Cyano oder C₁-C₂-Haloalkoxy steht,
R⁴ für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsilyl steht,
R⁵ für ein Pyrazol- oder Pyrrolring der Reihe R⁵-3 bis R⁵-8 steht, wobei jedes R⁵ mit R⁶ substituiert ist und gegebenenfalls mit R⁷ oder R⁸ oder sowohl R⁷ als auch R⁸ substituiert sein kann, 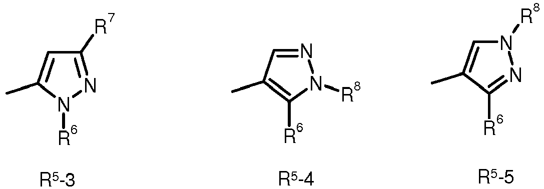 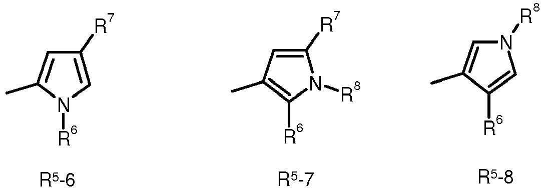
R⁶ für 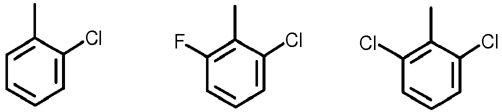 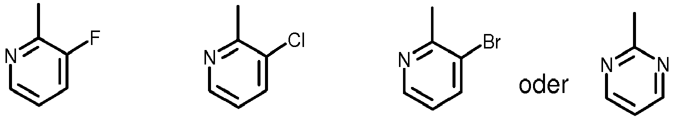 steht,
R⁷ für Wasserstoff, Halogen oder C₁-C₄-Haloalkyl steht,
R⁸ für Wasserstoff, C₁-C₄-Alkyl oder C₁-C₄-Haloalkyl steht,
sowie deren N-Oxide und Salze.

2. Verbindungen der Formel (II) 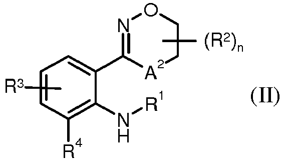 wobei R¹, R², R³, R⁴, A² und n die in Anspruch 1 angegebenen Bedeutungen haben.

3. Verbindungen der Formel (IV) 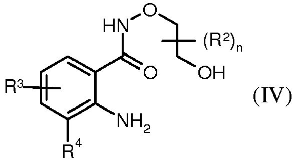 wobei R², R³, R⁴ und n die in Anspruch 1 angegebenen Bedeutungen haben,.

4. Verbindungen der Formel (VII) 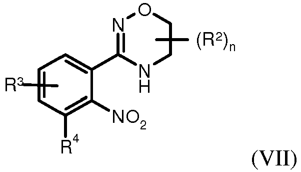 wobei R², R³, R⁴ und n die in Anspruch 1 angegebenen Bedeutungen haben.

5. Verbindungen der Formel (VIII) 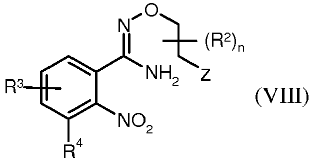 wobei R², R³, R⁴ und n die in Anspruch 1 angegebenen Bedeutungen haben und Z für Chlor, Brom, Iod, Methylsulfonyl oder Tolylsulfonyl steht

6. Verbindungen der Formel (IX) 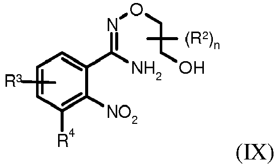 wobei R², R³, R⁴ und n die in Anspruch 1 angegebenen Bedeutungen haben.

7. Verfahren zur Herstellung von Verbindungen der Formel (I) gemäß Anspruch 1, **dadurch gekennzeichnet, dass** man
(A) Verbindungen der Formel (I-a) 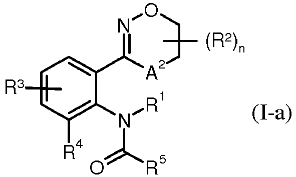 in welcher R¹, R², R³, R⁴, R⁵, A² und n die in Anspruch 1 angegebenen Bedeutungen haben, erhält, indem man Verbindungen der Formel (II) gemäß Anspruch 3 mit einem Säurechlorid der Formel (III) 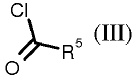 umsetzt,
und dass man
(B) Verbindungen der Formel (I-b) 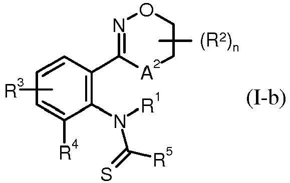 in welcher R¹, R², R³, R⁴, R⁵, A² und n die in Anspruch 1 angegebenen Bedeutungen haben, erhält, indem man Verbindungen der Formel (I-a) mit einem Schwefelungsreagenz umsetzt.

8. Verfahren zur Herstellung von Verbindungen der Formel (II) gemäß Anspruch 2, in welchen R¹ nicht Wasserstoff ist, **dadurch gekennzeichnet, dass** man Verbindungen der Formel (II), in denen R¹ Wasserstoff ist,
(A) mit einem Alkylierungsmittel in Gegenwart einer Base in Gegenwart eines Verdünnungsmittels umsetzt oder
(B) zuerst in einer Kondensationsreaktion in Gegenwart eines Verdünnungsmittels und anschließend mit einem Reduktionsmittel in Gegenwart eines Verdünnungsmittels umsetzt.

9. Verfahren zur Herstellung von Verbindungen der Formel (IV) gemäß Anspruch 3, **dadurch gekennzeichnet, dass** man Verbindungen der Formel (V) 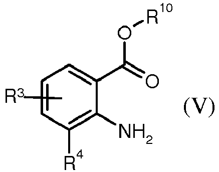 in welcher R³ und R⁴ die in Anspruch 1 angegegebenen Bedeutungen haben kann und R¹⁰ für C₁-C₄-Alkyl steht, mit Verbindungen der Formel (VI) 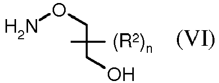 in welcher R² und n die in Anspruch 1 angegebenen Bedeutungen haben kann,
in Gegenwart einer Base sowie gegebenenfalls in Gegenwart eines Verdünnungsmittels umsetzt.

10. Verfahren zur Herstellung von Verbindungen der Formel (II-b) 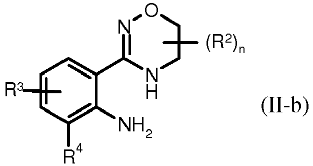 in welcher R², R³, R⁴ und n die in Anspruch 1 gegebenen Bedeutungen haben, **dadurch gekennzeichnet, dass** man Verbindungen der Formel (VII) 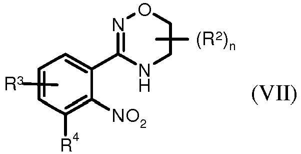 in der R², R³, R⁴ und n die in Anspruch 1 angegebenen Bedeutungen haben, mit einem Reduktionsmittel in Gegenwart eines Verdünnungsmittels umsetzt.

11. Verfahren zur Herstellung von Verbindungen der Formel (VII) gemäß Anspruch 4, **dadurch gekennzeichnet, dass** man Verbindungen der Formel (VIII) gemäß Anspruch 5, mit einer Base in Gegenwart eines Lösungsmittels umsetzt.

12. Verfahren zur Herstellung von Verbindungen der Formel (VIII) gemäß Anspruch 5, **dadurch gekennzeichnet, dass** man Verbindungen der Formel (IX) gemäß Anspruch 6 mit einem Sulfonylchlorid oder einem Halogenierungsmittel gegebenenfalls in Anwesenheit eines Lösungsmittels und gegebenenfalls in Anwesenheit einer Base umsetzt.

13. Verfahren zur Herstellung von Verbindungen der Formel (IX) gemäß Anspruch 6, **dadurch gekennzeichnet, dass** man Verbindungen der Formel (X) 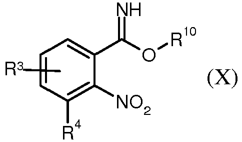 in welcher R³ und R⁴ die in Anspruch 1 angegebenen Bedeutungen haben und R¹⁰ für C₁-C₄-Alkyl steht, mit Verbindungen der Formel (VI) 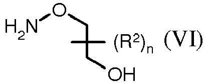 in welcher R² und n die in Anspruch 1 angegebenen Bedeutungen haben,
in Gegenwart eines anorganischen Salzes sowie in Gegenwart eines Verdünnungsmittels umsetzt.

14. Verfahren zur Herstellung von Verbindungen der Formel (II-b) gemäß Anspruch 10, in welcher R³ für 4-Chlor, 4-Brom oder 4-Iod steht, **dadurch gekennzeichnet, dass** man Verbindungen der allgemeinen Formel (II-b') 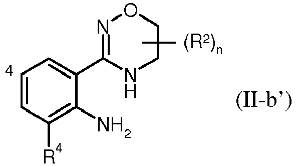 in welcher R², R⁴ und n die in Anspruch 1 angegebenen Bedeutungen haben, mit einem Halogenierungsmittel in Gegenwart eines Verdünnungsmittels umsetzt.

15. Verwendung von Verbindungen der Formel (I) gemäß Anspruch 1 zur Herstellung von Schädlingsbekämpfungsmitteln.

16. Verwendung von Verbindungen der Formel (I) gemäß Anspruch 1 zur Bekämpfung von tierischen Schädlingen- ausgenommen zur therapeutischen Behandlung des tierischen oder menschlichen Körpers.

17. Schädlingsbekämpfungsmittel, **gekennzeichnet durch** einen Gehalt an mindestens einer Verbindung der Formel (I) gemäß Anspruch 1.

18. Verfahren zur Bekämpfung von tierischen Schädlingen, ausgenommen zur therapeutischen Behandlung des tierischen oder menschlichen Körpers, **dadurch gekennzeichnet, dass** man Verbindungen der Formel (I) gemäß Anspruch 1 auf Schädlinge und/oder ihren Lebensraum einwirken lässt.

19. Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Stoffen vermischt.

20. Verwendung von Verbindungen der Formel (I) gemäß Anspruch 1 zur Behandlung von Saatgut.

21. Verwendung von Verbindungen der Formel (I) gemäß Anspruch 1 zur Behandlung von transgenen Pflanzen.

22. Verwendung von Verbindungen der Formel (I) gemäß Anspruch 1 zur Behandlung von Saatgut von transgenen Pflanzen.

23. Saatgut, umhüllt mit einer Verbindung der Formel (1) gemäß Anspruch 1.

## Claims

1. Compounds of the formula (I) 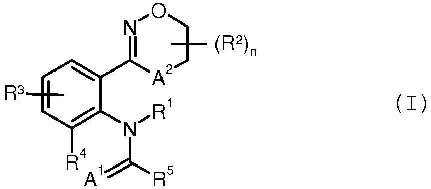 where
A¹ represents oxygen,
A² represents amino,
R¹ represents hydrogen, methyl, cyclopropyl, cyanomethyl, methoxymethyl, methylthio methyl, methylsulphinyl methyl or methylsulphonyl methyl,
R² independently of one another represent C₁-C₄-alkyl which is optionally mono- or polysubstituted by identical or different substituents, where the substituents independently of one another may be selected from the group consisting of halogen, cyano, nitro, C₁-C₄-alkoxy and C₁-C₄-haloalkoxy,
n represents 0,
R³ represents hydrogen, C₁-C₄-alkyl, C₁-C₂-haloalkyl, halogen, cyano or C₁-C₂-haloalkoxy,
R⁴ represents C₁-C₄-alkyl, C₃-C₆-CyCloalkyl, C₁-C₄-haloalkyl, C₁-C₆-halocycloalkyl, C₂-C₆-alkenyl, C₂-C₄-haloalkenyl, C₂-C₄-alkynyl, C₂-C₄-haloalkynyl, C₁-C₄-alkoxy, C₁-C₄-haloalkoxy, fluorine, chlorine, bromine, iodine, cyano, nitro or C₃-C₆-trialkylsilyl,
R⁵ represents a pyrazole or pyrrole ring of the group R⁵-3 to R⁵-8, where each R⁵ is substituted by R⁶ and may optionally be substituted by R⁷ or R⁸ or both R⁷ and R⁸, 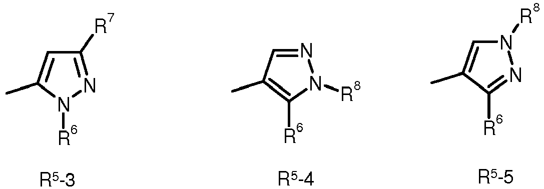 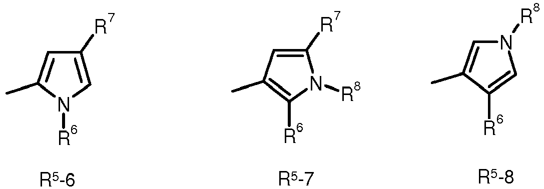
R⁶ represents 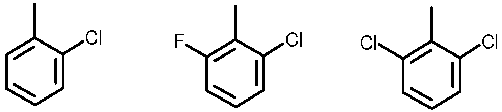 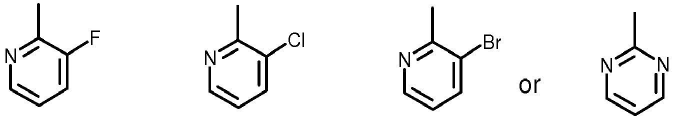
R⁷ represents hydrogen, halogen or C₁-C₄-haloalkyl,
R⁸ represents hydrogen, C₁-C₄-alkyl or C₁-C₄-haloalkyl,
and their N-oxides and salts.

2. Compounds of the formula (II) 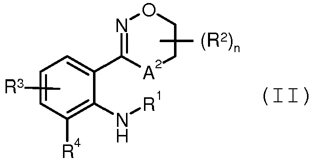 where R¹, R², R³, R⁴, A² and n are as defined in Claim 1.

3. Compounds of the formula (IV) 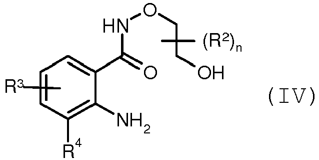 where R², R³, R⁴ and n are as defined in Claim 1.

4. Compounds of the formula (VII) 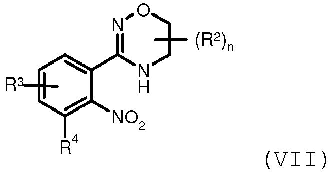 where R², R³, R⁴ and n are as defined in Claim 1.

5. Compounds of the formula (VIII) 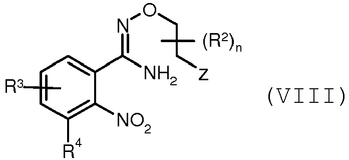 where R², R³, R⁴ and n are as defined in Claim 1 and Z represents chlorine, bromine, iodine, methylsulphonyl or tolylsulphonyl.

6. Compounds of the formula (IX) 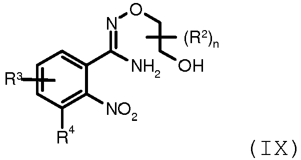 where R², R³, R⁴ and n are as defined in Claim 1.

7. Process for preparing compounds of the formula (I) according to Claim 1, **characterized in that**
(A) compounds of the formula (I-a) 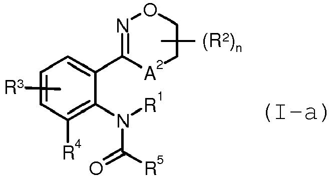 in which R¹, R², R³, R⁴, R⁵, A² and n are as defined in Claim 1 are obtained by reacting compounds of the formula (II) according to Claim 3 with an acid chloride of the formula (III) 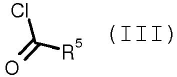 and that
(B) compounds of the formula (I-b) 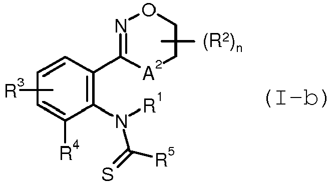 in which R¹, R², R³, R⁴, R⁵, A² and n are as defined in Claim 1 are obtained by reacting compounds of the formula (I-a) with a sulphurizing agent.

8. Process for preparing compounds of the formula (II) according to Claim 2 in which R¹ does not represent hydrogen, **characterized in that** compounds of the formula (II) in which R¹ is hydrogen
(A) are reacted with an alkylating agent in the presence of a base in the presence of a diluent or
(B) are reacted initially in a condensation reaction in the presence of a diluent and then with a reducing agent in the presence of a diluent.

9. Process for preparing compounds of the formula (IV) according to Claim 3, **characterized in that** compounds of the formula (V) 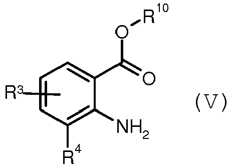 in which R³ and R⁴ are as defined in Claim 1 and R¹⁰ represents C₁-C₄-alkyl are reacted with compounds of the formula (VI) 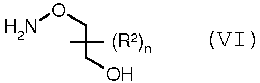 in which R² and n are as defined in Claim 1,
in the presence of a base and, if appropriate, in the presence of a diluent.

10. Process for preparing compounds of the formula (II-b) 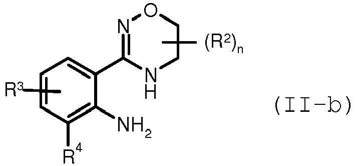 in which R², R³, R⁴ and n are as defined in Claim 1, **characterized in that** compounds of the formula (VII) 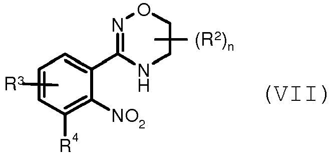 in which R², R³, R⁴ and n are as defined in Claim 1 are reacted with a reducing agent in the presence of a diluent.

11. Process for preparing compounds of the formula (VII) according to Claim 4, **characterized in that** compounds of the formula (VIII) according to Claim 5 are reacted with a base in the presence of a solvent.

12. Process for preparing compounds of the formula (VIII) according to Claim 5, **characterized in that** compounds of the formula (IX) according to Claim 6 are reacted with a sulphonyl chloride or a halogenating agent, if appropriate in the presence of a solvent and if appropriate in the presence of a base.

13. Process for preparing compounds of the formula (IX) according to Claim 6, **characterized in that** compounds of the formula (X) 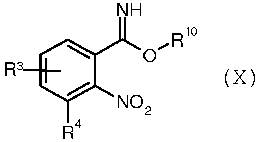 in which R³ and R⁴ are as defined in Claim 1 and R¹⁰ represents C₁-C₄-alkyl are reacted with compounds of the formula (VI) 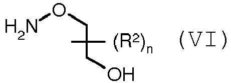 in which R² and n are as defined in Claim 1,
in the presence of an inorganic salt and in the presence of a diluent.

14. Process for preparing compounds of the formula (II-b) according to Claim 10 in which R³ represents 4-chloro, 4-bromo or 4-iodo, **characterized in that** compounds of the general formula (II-b') 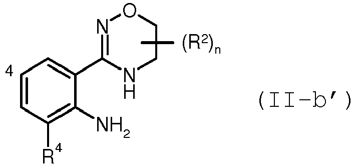 in which R², R⁴ and n are as defined in Claim 1 are reacted with a halogenating agent in the presence of a diluent.

15. Use of compounds of the formula (I) according to Claim 1 for preparing pesticides.

16. Use of compounds of the formula (I) according to Claim 1 for controlling animal pests, except for the therapeutic treatment of the animal or human body.

17. Pesticides, **characterized in that** they comprise at least one compound of the formula (I) according to Claim 1.

18. Method for controlling animal pests, except for the therapeutic treatment of the animal or human body, **characterized in that** compounds of the formula (I) according to Claim 1 are allowed to act on pests and/or their habitat.

19. Process for preparing pesticides, **characterized in that** compounds of the formula (I) according to Claim 1 are mixed with extenders and/or surfactants.

20. Use of compounds of the formula (I) according to Claim 1 for treating seed.

21. Use of compounds of the formula (I) according to Claim 1 for treating transgenic plants.

22. Use of compounds of the formula (I) according to Claim 1 for treating seed of transgenic plants.

23. Seed coated with a compound of the formula (I) according to Claim 1.

## Revendications

1. Composés de formule (I) 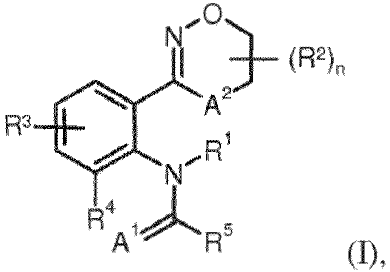 dans laquelle
A¹ représente oxygène,
A² représente amino,
R¹ représente hydrogène, méthyle, cyclopropyle, cyanométhyle, méthoxyméthyle, méthylthiométhyle, méthylsulfinylméthyle ou méthylsulfonylméthyle,
les R² représentent indépendamment les uns des autres alkyle en C₁-C₄ éventuellement substitué une ou plusieurs fois, de manière identique ou différente, les substituants pouvant être choisis indépendamment les uns des autres parmi halogène, cyano, nitro, alcoxy en C₁-C₄ et haloalcoxy en C₁-C₄,
n représente 0,
R³ représente hydrogène, alkyle en C₁-C₄, haloalkyle en C₁-C₂, halogène, cyano ou haloalcoxy en C₁-C₂,
R⁴ représente alkyle en C₁-C₄, cycloalkyle en C₃-C₆, haloalkyle en C₁-C₄, halocycloalkyle en C₁-C₆, alcényle en C₂-C₆, haloalcényle en C₂-C₄, alcynyle en C₂-C₄, haloalcynyle en C₂-C₄, alcoxy en C₁-C₄, haloalcoxy en C₁-C₄, fluor, chlore, brome, iode, cyano, nitro ou trialkylsilyle en C₃-C₆,
R⁵ représente un cycle pyrazole ou pyrrole de la série R⁵-3 à R⁵-8, chaque R⁵ étant substitué avec R⁶ et pouvant éventuellement être substitué avec R⁷ ou R⁸ ou aussi bien R⁷ que R⁸, 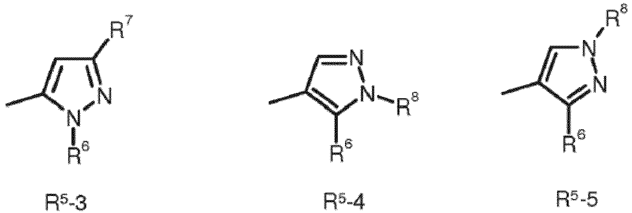 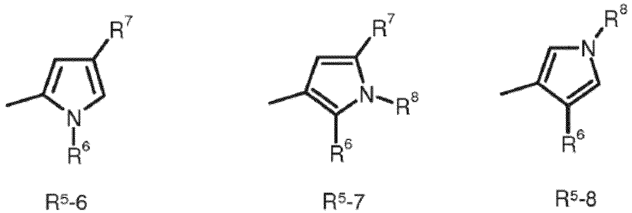
R⁶ représente 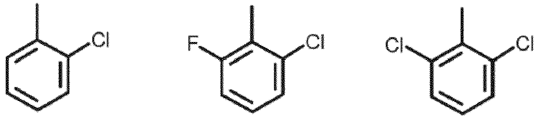 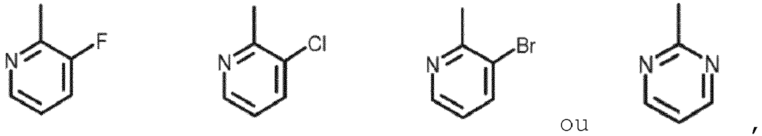
R⁷ représente hydrogène, halogène ou haloalkyle en C₁-C₄,
R⁸ représente hydrogène, alkyle en C₁-C₄ ou haloalkyle en C₁-C₄,
ainsi que leurs N-oxydes et sels.

2. Composés de formule (II) 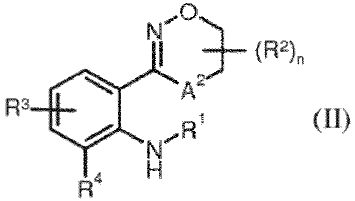 dans laquelle R¹, R², R³, R⁴, A² et n ont les significations indiquées dans la revendication 1.

3. Composés de formule (IV) 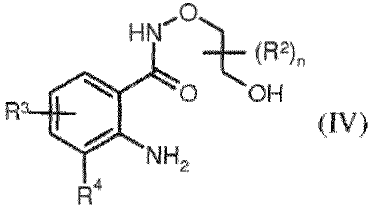 dans laquelle R², R³, R⁴ et n ont les significations indiquées dans la revendication 1.

4. Composés de formule (VII) 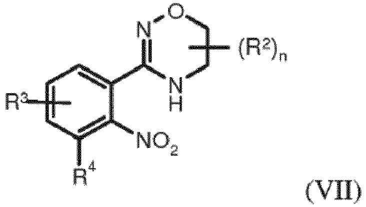 dans laquelle R², R³, R⁴ et n ont les significations indiquées dans la revendication 1.

5. Composés de formule (VIII) 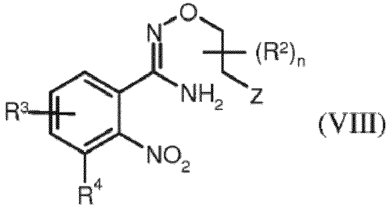 dans laquelle R², R³, R⁴ et n ont les significations indiquées dans la revendication 1 et Z représente chlore, brome, iode, méthylsulfonyle ou tolylsulfonyle.

6. Composés de formule (IX) 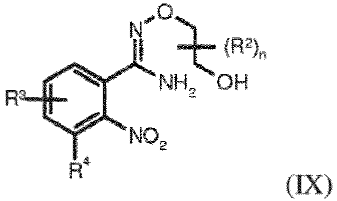 dans laquelle R², R³, R⁴ et n ont les significations indiquées dans la revendication 1.

7. Procédé de fabrication de composés de formule (I) selon la revendication 1, **caractérisé en ce que**
(A) des composés de formule (I-a) 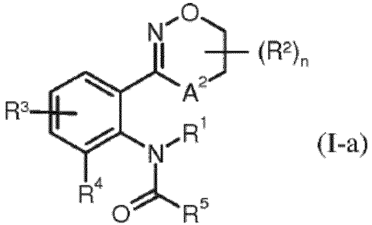 dans laquelle R¹, R², R³, R⁴, R⁵, A² et n ont les significations indiquées dans la revendication 1, sont obtenus par mise en réaction de composés de formule (II) selon la revendication 3 avec un chlorure d'acide de formule (III) 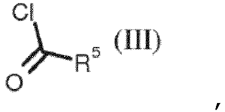 et **en ce que**
(B) des composés de formule (I-b) 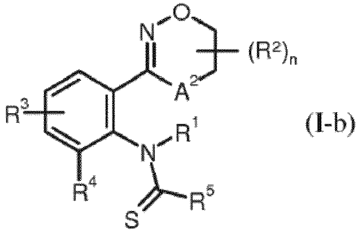 dans laquelle R¹, R², R³, R⁴, R⁵, A² et n ont les significations indiquées dans la revendication 1, sont obtenus par mise en réaction de composés de formule (I-a) avec un réactif de sulfuration.

8. Procédé de fabrication de composés de formule (II) selon la revendication 2, dans lesquels R¹ ne représente pas l'hydrogène, **caractérisé en ce que** des composés de formule (II) dans lesquels R¹ représente l'hydrogène,
(A) sont mis en réaction avec un agent d'alkylation en présence d'une base et en présence d'un diluant, ou
(B) sont tout d'abord mis en réaction par une réaction de condensation en présence d'un diluant, puis avec un réducteur en présence d'un diluant.

9. Procédé de fabrication de composés de formule (IV) selon la revendication 3, **caractérisé en ce que** des composés de formule (V) 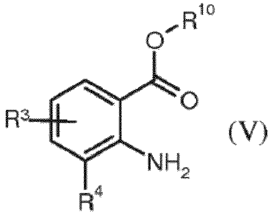 dans laquelle R³ et R⁴ peuvent avoir les significations indiquées dans la revendication 1, et R¹⁰ représente alkyle en C₁-C₄, sont mis en réaction avec des composés de formule (VI) 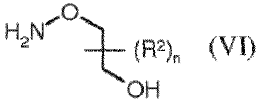 dans laquelle R² et n peuvent avoir les significations indiquées dans la revendication 1,
en présence d'une base et éventuellement en présence d'un diluant.

10. Procédé de fabrication de composés de formule (II-b) 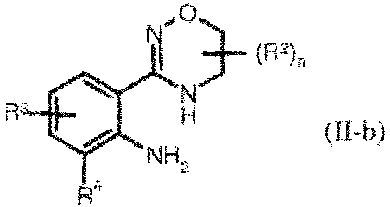 dans laquelle R², R³, R⁴ et n ont les significations indiquées dans la revendication 1, **caractérisé en ce que** des composés de formule (VII) 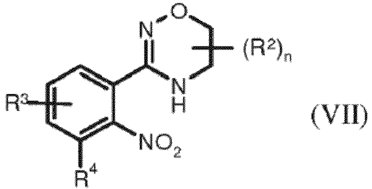 dans laquelle R², R³, R⁴ et n ont les significations indiquées dans la revendication 1, sont mis en réaction avec un réducteur en présence d'un diluant.

11. Procédé de fabrication de composés de formule (VII) selon la revendication 4, **caractérisé en ce que** des composés de formule (VIII) selon la revendication 5 sont mis en réaction avec une base en présence d'un solvant.

12. Procédé de fabrication de composés de formule (VIII) selon la revendication 5, **caractérisé en ce que** des composés de formule (IX) selon la revendication 6 sont mis en réaction avec un chlorure de sulfonyle ou un agent d'halogénation, éventuellement en présence d'un solvant et éventuellement en présence d'une base.

13. Procédé de fabrication de composés de formule (IX) selon la revendication 6, **caractérisé en ce que** des composés de formule (X) 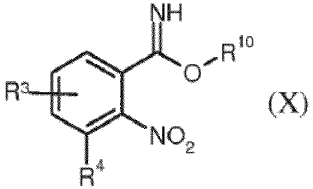 dans laquelle R³ et R⁴ ont les significations indiquées dans la revendication 1 et R¹⁰ représente alkyle en C₁-C₄, sont mis en réaction avec des composés de formule (VI) 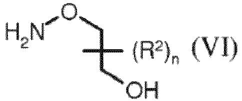 dans laquelle R² et n ont les significations indiquées dans la revendication 1,
en présence d'un sel inorganique et en présence d'un diluant.

14. Procédé de fabrication de composés de formule (II-b) selon la revendication 10, dans lesquels R³ représente 4-chlore, 4-brome ou 4-iode, **caractérisé en ce que** des composés de formule général (II-b') 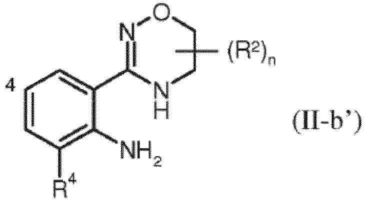 dans laquelle R², R⁴ et n ont les significations indiquées dans la revendication 1, sont mis en réaction avec un agent d'halogénation en présence d'un diluant.

15. Utilisation de composés de formule (I) selon la revendication 1 pour la fabrication d'agents de lutte contre des nuisibles.

16. Utilisation de composés de formule (I) selon la revendication 1 pour lutter contre des animaux nuisibles, à l'exception du traitement thérapeutique du corps animal ou humain.

17. Agent de lutte contre des nuisibles, **caractérisé par** une teneur en au moins un composé de formule (I) selon la revendication 1.

18. Procédé de lutte contre des animaux nuisibles, à l'exception du traitement thérapeutique du corps animal ou humain, **caractérisé en ce que** des composés de formule (I) selon la revendication 1 sont laissés agir sur des nuisibles et/ou leur habitat.

19. Procédé de fabrication d'agents de lutte contre des nuisibles, **caractérisé en ce que** des composés de formule (I) selon la revendication 1 sont mélangés avec des extendeurs et/ou des substances tensioactives.

20. Utilisation de composés de formule (I) selon la revendication 1 pour le traitement de graines.

21. Utilisation de composés de formule (I) selon la revendication 1 pour le traitement de plantes transgéniques.

22. Utilisation de composés de formule (I) selon la revendication 1 pour le traitement de graines de plantes transgéniques.

23. Graines, enrobées avec un composé de formule (I) selon la revendication 1.
